Europäisches Patentamt

European Patent Office

Office européen des brevets

(11) Publication number: **0 205 625**
A1

# EUROPEAN PATENT APPLICATION

published in accordance with Art. 158(3) EPC

(21) Application number: 86900287.3

(22) Date of filing: 28.12.85

Data of the international application taken as a basis:

(86) International application number: PCT/JP 85/00733

(87) International publication number: WO 86/03975 (17.07.86 86/17)

(51) Int. Cl.⁴: **A 61 K 39/29**, C 07 K 15/04, C 12 P 21/00

(30) Priority: 28.12.84 JP 274592/84

(43) Date of publication of application: 30.12.86 Bulletin 86/52

(84) Designated Contracting States: FR

(71) Applicant: JAPANESE FOUNDATION FOR CANCER RESEARCH, 37-1, Kamiikebukuro 1-chome, Toshima-ku Tokyo (JP)
Applicant: MEIJI MILK PRODUCTS COMPANY LIMITED, 3-6, Kyobashi 2-chome Chuo-ku, Tokyo 104 (JP)

(72) Inventor: KOIKE, Katsuo, 14-31, Mure 4-chome, Mitaka-shi Tokyo 181 (JP)
Inventor: SUGANO, Haruo, 8-13, Minamiogikubo 4-chome, Suginami-ku Tokyo 167 (JP)
Inventor: ODA, Munehiro, 21-3, Igusa 1-chome, Suginami-ku Tokyo 167 (JP)
Inventor: KATAYANAGI, Shigeru, Daiichi-Seiryuso 114 1123, Kayama, Odawara-shi Kanagawa-ken 250-01 (JP)
Inventor: FUJIMORI, Takao, 3859-10, Akuwa-cho Seya-ku, Yokohama-shi Kanagawa-ken 246 (JP)

(74) Representative: Kohn, Armand, 5 Avenue Foch, F-92380 Garches (FR)

(54) HEPATITIS B VIRUS VACCINE AND PROCESS FOR ITS PREPARATION.

(57) A composition of polypeptides (and/or glycopolypeptides wherein a sugar chain is bound to said polypeptide) composed of one or more of an adr-type hepatitis B virus surface antigen and/or intermediate surface antigen of said and/or large surface antigen of said virus, a process for its preparation, and a vaccine for hepatitis B virus containing said composition as an effective ingredient. The vaccine can be produced in a large amount by culturing variant strain huGK-14, thus being advantageously produced without resort to human blood.

**TITLE MODIFIED**
see front page

HEPATITIS B VACCINE AND PROCESS FOR

PRODUCTION OF THE SAME

BACKGROUND OF THE INVENTION

1. Field of the Invention

This invention relates to a composition which comprises at least one polypeptide and/or glycopolypeptide having sugar chains attached thereto, of adr hepatitis B virus small surface antigen and/or said virus middle surface antigen and/or said virus large surface antigen, a process for the production of the same and a vaccine for preventing hepatitis B containing the same as an active ingredient.

2. Description of the Prior Art

Hepatitis B is a viral disease frequently observed in the Far East including Japan, Southeast Asia and Africa. It is also detected in Europe and North America but the incidence thereof in these areas is relatively low. This disease is caused by hepatitis B virus (HBV) which is classified into four subtypes, i.e., adw, adr, ayw and ayr, depending on the antigenic determinants. Among these subtypes, adr and adw are detected in Japan and particularly the former is frequently observed therein. On the other hand, ayw and adw are dominant subtypes in Europe and North America.

Recent biological, molecular biological and immunological studies have gradually clarified the structure and function of HBV. Spherical particles called Dane particles of 42 nm in diameter which have a complete DNA virus structure, smaller spherical particles of approximately 22 nm in diameter and tubular particles are observed in the blood of an infected person. A Dane particle carries surface antigen (HBsAg) in the lipid-containing envelope and core antigen (HBcAg), e antigen (HBeAg) as well as long- and short-chain DNA carrying genetic information and DNA polymerase therein.

Many attempts have been made to produce a vaccine by purifying HBsAg since 1970 because an antibody against a virus surface antigen might be effective in preventing the infection by said virus (cf. references 1 to 4). Further studies on an amino acid sequence (pre-S) attached to the N-terminal of Hepatitis B small surface antigen have been carried out since 1982 because it might enhance the effect of the vaccine. Further attempts have been made to develop vaccines of HBcAg and HBeAg for non-sensitive subjects including those receiving dialysis (cf. reference 5).

Until 1980, these studies had been performed mainly by purifying HBsAg from the blood of an infected person. Following U.S.A. and France, HBsAg originating from human blood was approved as a vaccine for preventing hepatitis B in 1984 in Japan (cf. reference 6). Based on the progress in molecular biology, recombinant DNA technology has come to be dominantly used in the study and development of HBsAg and HBsAg having pre-S since 1980. Consequently the whole primary DNA structures of three subtypes (ayw, adw and adr) among four have been already determined (cf. references 7 to 9 and 20).

However it has been very difficult to produce HBsAg with or without pre-S in such a large amount as practically required as a vaccine. No animal nor cultured cell but only man and chimpanzee can be infected by HVB. Thus HBsAg is usually isolated and purified from the blood of a human carrier. However the amount of HBsAg collected from human carriers is strictly limited and hardly meets the demand. In addition, there is a risk of contamination of impurities and viruses from the blood.

Under these circumstances, many attempts have been made to produce desired antigens by transformation of Escherichia coli or yeasts as a host through recombinant DNA technology. The results of these studies have been frequently reported (cf. references 10 to 14), while at the same time, new problems have been revealed. For example, it has been reported that the expression of a DNA coding for HBsAg in most hosts is significantly limited (cf.

- 4 -

0205625

reference 13). In the case of <u>Escherichia coli</u> as a host, HBsAg is liable to breakdown in host cells and sometimes inhibits the growth of <u>E</u>. <u>coli</u> (cf. reference 14). In the case of yeasts as a host, the production of HBsAg in some yeasts is also limited and the purification of HBsAg is too difficult to obtain a satisfactory amount. Furthermore it has been reported that HBsAg extracted from <u>E</u>. <u>coli</u> or yeast cells has a low immunogenicity (cf. references 15 and 16).

It was at first understood that yeasts were the most suitable hosts in the priduction of antigens through recombinant DNA technology. However it is now understood that the low immunogenicities of the antigens thus obtained suggest that their activities considerably depend on the hosts. For example, the presence of sugar chains is considered to affect the immunogenicity. Thus some studies including an infection method into mammal cells and an expression method using an expression vector containing a DNA coding for an antigenic polypeptide at various phases of the culture (cf. reference 17) and another method wherein a DNA coding for an antigenic polypeptide is integrated into mammal cells to thereby induce expression (cf. reference 18) have attracted attention, although the number of these studies is limited. The former process is troublesome and gives a low productivity of HBsAg. While the latter process has disadvantages in that the maintenence of the recombinant is difficult and that the amount of HBsAg thus produced hardly meets the demand (cf. references 17 and 18). In addition, each process required a considerable amount of serum for culturing cells, which makes the performance of the same at a large scale too expensive.

BRIEF DESCRIPTION OF THE DRAWINGS

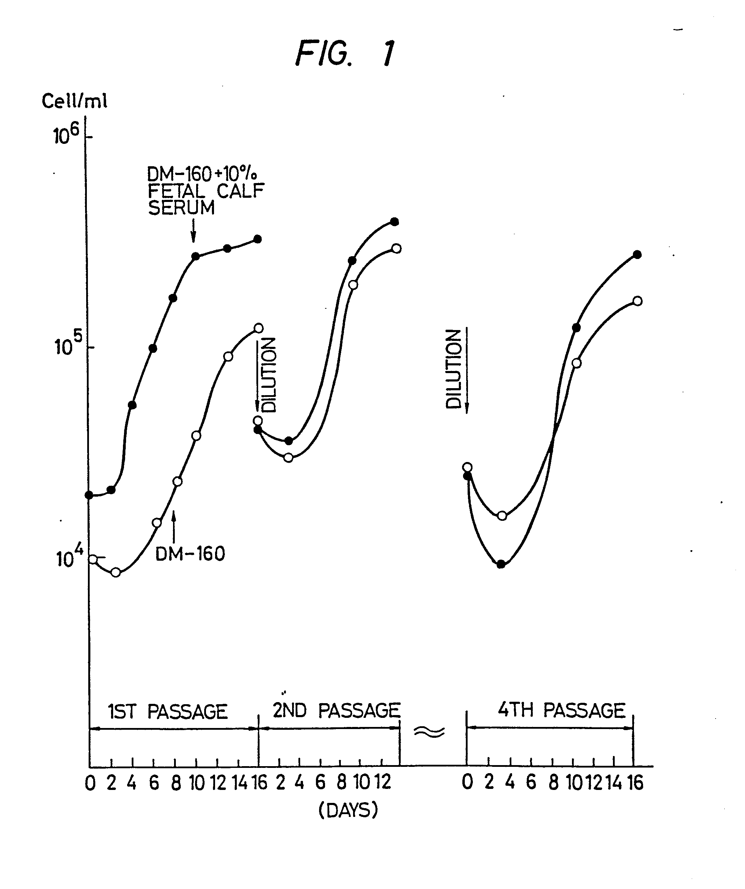

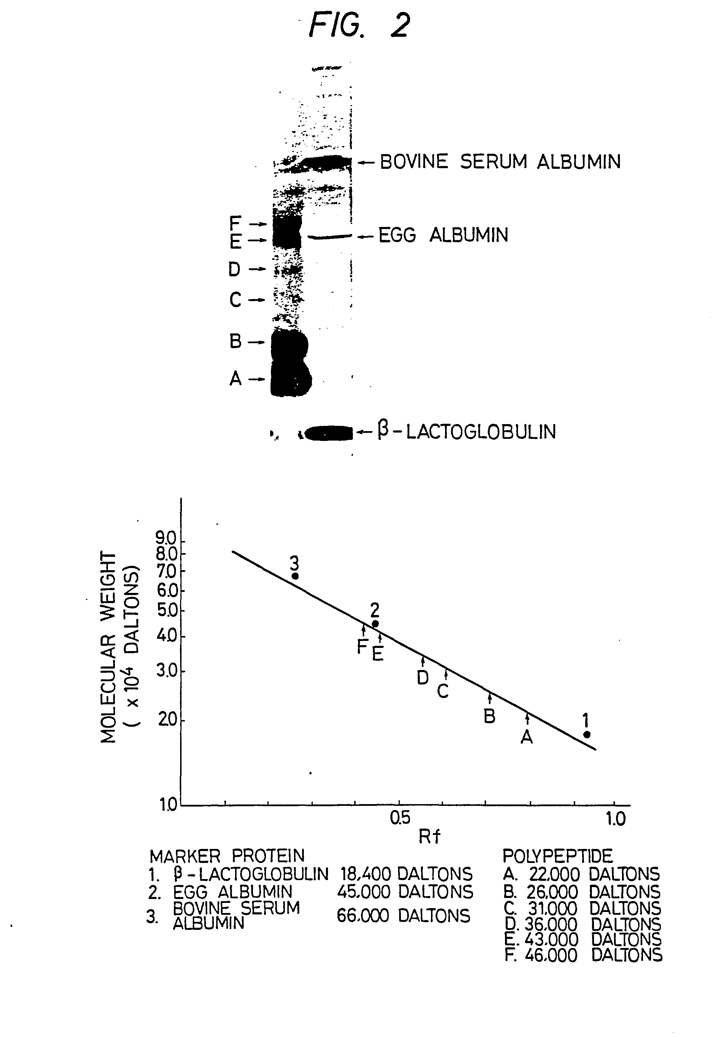

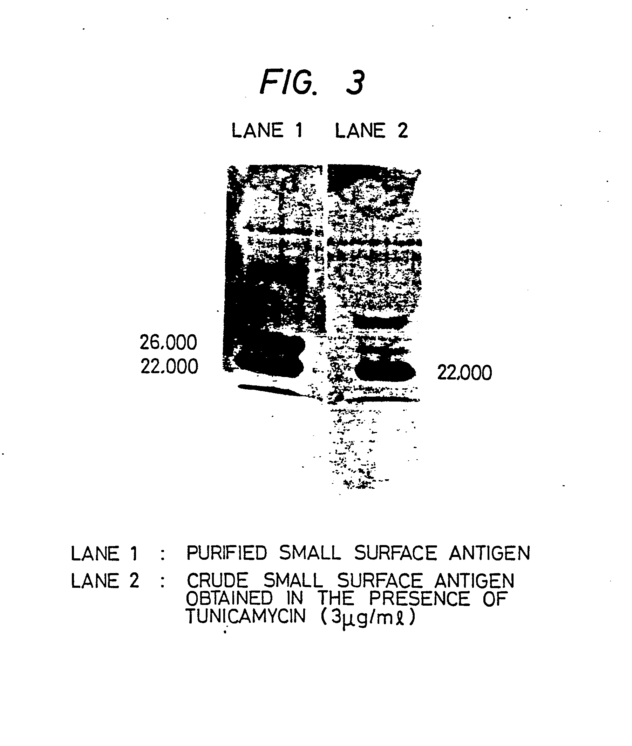

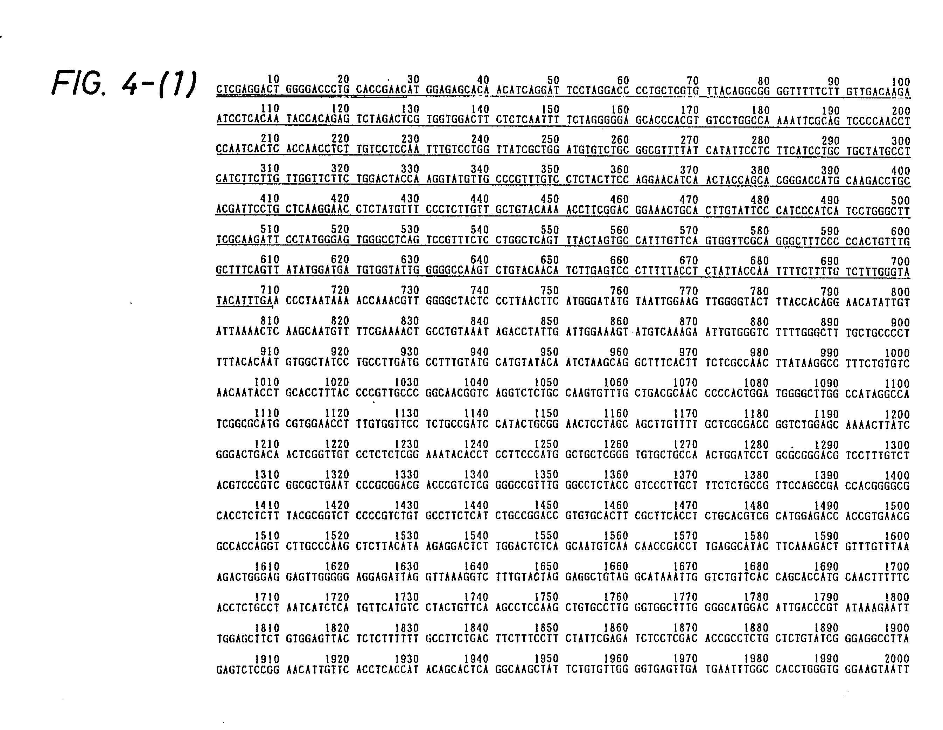

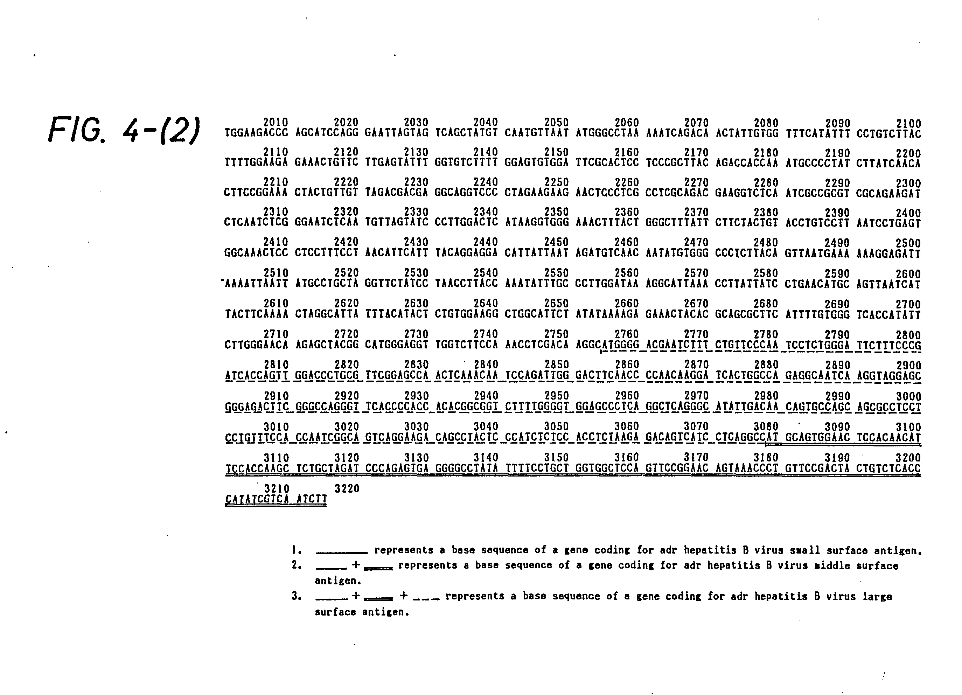

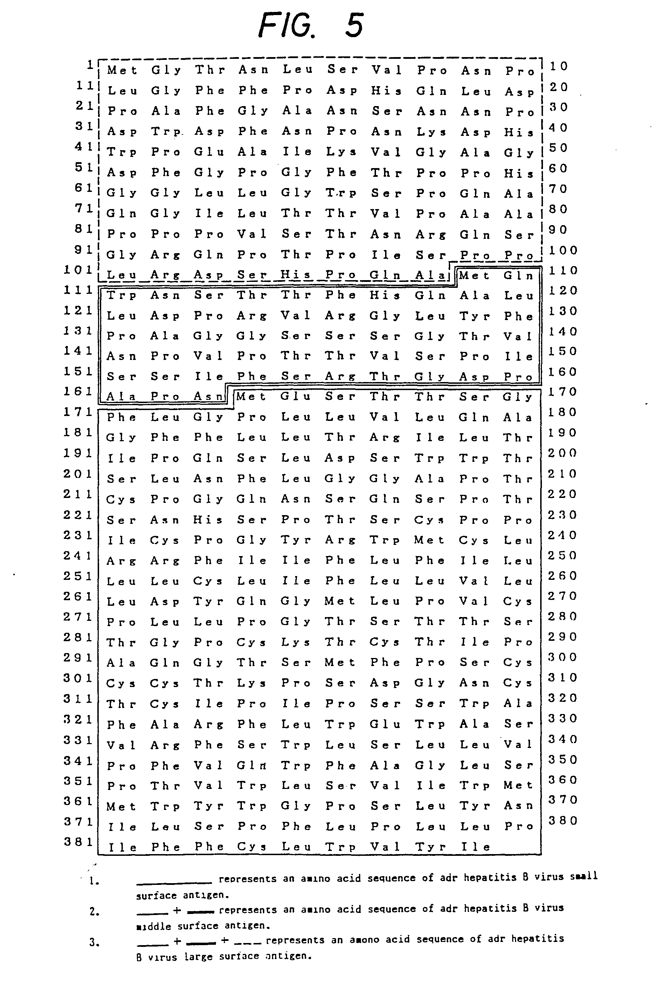

Fig. 1 shows the result of subcultivation of huGK-14 in serum-containing and serum-free media. Fig. 2 shows the result of SDS polyacrylamide gel electrophoresis of adr hepatitis B virus small surface antigen, as well as those of said virus middle surface antigen and said virus large surface antige, each produced by huGK-14. Fig. 3 shows the result of SDS polyacrylamide gel electrophoresis of adr hepatitis B virus small surface antigen obtained by the cultivation in the presence of tunicamycin. Fig. 4 shows an example of the whole primary DNA structure of adr hepatitis B virus small surface antigen, as well as those of said virus middle surface antigen and said virus large surface antigen. Fig. 5 shows an example of an amino acid sequence of adr hepatitis B virus small surface antigen, as well as those of said virus middle surface antigen and said virus large surface antigen.

DETAILED DESCRIPTION OF THE INVENTION

Through extensive studies aiming at overcoming these disadvantages and producing a large amount of an antigen effective as a vaccine for preventing hepatitis B, we have succeeded in cloning from human hepatoma cells a variant cell strain huGK-14 which carries DNA fragments of adr HBV and produces a large amount of an antigen composition comprising HBsAg and those to which pre-S is attached. The variant cell strain huGK-14 has been established by repeated selection of colonies of huSP, which is a human hepatoma cell strain previously established by Koike at al. (cf. reference 19), showing a high productivity of HBsAg, followed by repeated cloning to thereby give a cell strain which shows a significantly higher HBsAg productivity than huSP and can be cultured in a serum-free medium, in contrast to the high serum requirement of huSP. These two cell strains are obviously different from each other from a biological viewpoint. Serum requirement of huGK-14 is demonstrated in the following test.

Serum-free and serum-containing DM-160 media were employed for comparison. Each medium was inoculated with huGK-14 to give a density of $1 \times 10^4$ cells/cm$^2$ to $4 \times 10^4$ cells/cm$^2$ on a plate having 24 wells. Then the cells were allowed to grow and treated with trypsin in a conventional manner at the point when the cell density reached saturation. Then the cells were inoculated to a fresh plate. Then this subculture was repeated at an interval of 14 to 16 days. Fig. 1 shows the result.

Thus it has been proved that huGK-14 can be cultured in a serum-free medium and shows proliferation even in repeated subculture.

huGK-14 can be nearly permanently stored in a frozen condition at -196°C. It has been deposited with the Cancer Institute, Japanese Foundation for Cancer Research and may be distributed if required.

HBsAg antigenic substance may be obtained from the culture supernatant obtained by culturing this cell strain. This HBsAg antigenic substance is isolated, purified and subjected to SDS-polyacrylamide gel electrophoresis. Thus six bands of different molecular weights are observed. These bands are classified into three pairs wherein the major one corresponds to molecular weights of 22,000 and 26,000 while the relatively minor two correspond to 31,000 and 36,000 and 43,000 and 46,000, respectively (cf. Fig. 2). In each pair, the two polypeptides differ from each other depending on the presence of sugar chains. The surface antigens isolated and purifed from a medium containing 3 μg/ml of tunicamycin, which serves as an inhibitor for sugar chain formation, give only three bands each corresponding to the respective lower molecular weights of the three pairs. This fact suggests that the polypeptide of the higher molecular weight in each pair corresponds to the polypeptide to which sugar chains are attached. As shown in Fig. 3, the bands of molecular weights of 22,000 and 26,000 are converted into a single band of 22,000.

It is considered that the two bands of molecular weights of 22,000 and 26,000 correspond to the small surface antigen, those of 31,000 and 36,000 correspond to the middle surface antigen and those of 43,000 and 46,000 correspond to the large surface antigen. The nucleotidic sequence coding for the surface antigen was detected by examining the major mRNA isolated from huGK-14 cells, which also proves that the major bands correspond to the small surface antigen. The binding properties with pre-S antibody (anti-pre-S antibody) have proved that the substances corresponding to the molecular weights of 31,000 and 36,000 and 43,000 and 46,000 are surface antigens in which pre-S is contained (cf. Table 4 in Example 1). Fig. 4 shows the whole primary DNA structure of adr hepatitis B virus determined by Koike et al., who are among the inventors of the present invention (cf. reference 20). The molecular weight of 22,000 is closely similar to that (25,363) of the polypeptide calculated from the DNA sequence coding for the small surface antigen. The molecular weight of 31,000 is roughly equal to that (31,163) of the polypeptide calcuated from the base sequence of the middle surface antigen wherein a DNA coding for 55 amino acids is attached to the 5' terminal side of the DNA coding for the small surface antigen. The molecular weight of 43,000 is roughly equal to that (42,565) of the polypeptide calculated from the base sequence of the large surface antigen wherein a DNA coding for 163 amino acids is attached to the 5' terminal side of the DNA coding for the small surface antigen (cf. Fig. 5). The 55 and/or 163 amino acids attached to the N-terminal are generally called pre-S.

It has been known that the adr hepatitis B virus DNA sequence has further variations. These variations are brought about not only by differences of the combination of three bases corresponding to an amino acid but by differences in several amino acids coded by the DNA (cf. references 21 and 22). The amino acid sequence of the small surface antigen of Fig. 5 differs from the one shown in reference 21 in the third, 165th and 184th amino acids counted from the initial amino acid coded by the initiation codon, corresponding to the 166th, 328th and 347th amino acids in Fig. 5. On the other hand, the amino acid sequence of the small surface antigen of Fig. 5 differs from the one shown in reference 22 in the third and 184th amino acids counted from the initial amino acid coded by the initiation codon, corresponding to the 166th and 347th amino acids in Fig. 5. Recent studies suggest that the amino acid sequence playing a role of an antigenic determinant of the small surface antigen may be present between the 110th and 156th amino acids counted from the initial amino acid coded by the initiation codon. Thus the abovementioned differences in the several amino acids may exert no effect on the antigenecity. Similar differences in several amino acids are observed in the amino acid sequence of the pre-S attached to the N-terminal side. However these differences are indistinguishable so long as the biological and immunological activities as small surface antigen with or without pre-S are the same.

It is possible to separate the polypeptide and glycopolypeptide of the small surface antigen and those of the one to which amino acids (pre-S) are attached at the N-terminal side by using anti-pre-S antibody if required (cf. references 28 and 24).

The composition comprising this small surface antigen and/or the one with pre-S may be readily isolated and purified from a culture supernatant of a serum-free medium to give a high purity. Since the host is a cell strain originating from human, a considerably high vaccine effect, particularly due to the presence of sugar chains derived from a human cell may be expected.

We have employed a variant cell strain huGK-14 originating from human hepatoma in order to produce a composition which comprises at least one polypeptide and/or glycopolypeptide of adr hepatitis B virus small surface antigen and/or said virus middle surface antigen and/or said virus large surface antigen. The biological properties of the cell strain are as follows.

1. Cell strain huGK-14 originating from human hepatoma

(1) It is a variant cell strain originating from human hepatoma.

(2) The number of its chromosomal modes is 51.

(3) Approximately eight DNA fragments carrying a gene coding for adr hepatitis B virus surface antigen are integrated in each chromosomal haploid of the same.

(4) It produces a large amount of a composition comprising adr hepatitis B virus small surface antigen, said virus middle surface antigen and said virus large surface antigen.

(5) The cell is in the form of a pseudomorphic circle and epithelial.

(6) It is adhesive.

(7) It grows best at 36.5 ± 1°C.

(8) The production of the antigens shows a rapid increase after the cell density reaches saturation.

(9) A DM-160 medium containing 4 % or more serum is suitable for its growth.

(10) It exhibits excellent proliferation and growth in a serum-free medium.

(11) A Williams' E medium is the most suitable for maintaining the viability after the cell density reaches saturation. Antigens may be recovered for a prolonged period by simply replacing the medium.

(12) The addition of dexamethasone can stimulate the production of the composition which comprises adr hepatitis B virus small surface antigen, said virus middle surface antigen and said virus large surface antigen.

The cell strain huGK-14 is obviously different from huSP (cf. references 19 and 23).

2. Procedure for cell culture

The cell strain huGK-14 is initially cultured in Petri dishes and then transferred into a culturing device suitable for mass culture such as "Cell Factory" (mfd. by Nunc Co., Ltd.) or a microcarrier when an appropriate number of cells are obtained, followed by adhesive culture therein. huGK-14 may exhibit proliferation in a basal medium mainly comprising amino acids. However it is preferable to grow it in a serum-containing medium and transplant the same into a serum-free medium immediately before the growth of the cells reaches the stationary phase in order to efficiently produce polypeptides and glycopolypeptides behaving as antigens to HBV. Production of the antigens is hardly observed in the growth phase and shows a rapid increase after the cell density reaches saturation. Thus a DM-160 medium (mfd. by Kyokuto Seiyaku Co., Ltd.) containing at least 4 % of fetal calf serum is suitable for the initial growth in Petri dishes and the succeeding growth phase in a mass culturing device. The medium is preferably replaced at an interval of two to three days. In the production phase in the mass culturing device, a Williams' E medium (mfg. by Flow Laboratories) containing dexamethasone, which is an antigen production inducer, as well as a cell adhesion factor such as fibronectin (mfd. by Sigma Co.) is employed. The culture is carried out for three months or longer with replacing the medium at an interval of six days to give the products. The addition of dexamethasone significantly promotes the production of the antigens compared with the case wherein no dexamethasone is added. Dexamethasone is preferably added at an amount of approximately $10^{-6}$ M. The culture temperature and atmospheric conditions are not strictly limited either in the growth phase or in the production phase. It is preferable to culture at 36.5 ± 1°C and at a $CO_2$ concentration of approximately 5 %.

The amount of the antigens produced by huGK-14 in the optimum medium is as follows. This value includes surface antigens with pre-S.

| Cell strain | HBsAg productivity | Medium |
|---|---|---|
| huGK-14 | 2,400 ng/ml | Williams' E medium + dexamethasone |

The effect of dexamethasone as an antigen production promotor can be determined by the following test. The product includes surface antigens with pre-S. Dexamethasone is added to give a concentration of $10^{-6}$ M. Table 1 shows the result obtained in the absence and presence of dexamethasone.

Table 1

| Medium | Production (ng/ml) |
|---|---|
| Williams' E containing no dexamethasone | 1,340 |
| Williams' E containing dexamethasone | 2,400 |

3. Purificaiton

The small surface antigen, middle surface antigen and large surface antigen recovered from the cell culture supernatant are purified by a combination of purifying procedures such as ultrafiltration, affinity chromatography and centrifugation. Among these procedures, affinity chromatography with the use of monoclonal antibodies may give extremely high purity and yield. The purification may be carried out in the following manner.

The culture supernatant is concentrated 10 to 30-fold with an ultrafiltration membrane (mfd. by Amicon Co., Ltd.) and subjected to a column to which anti-HBsAg monoclonal antibodies bind. The monoclonal antibodies are prepared by K.K. Shino-test Laboratory and bind to either the adr hepatitis B virus small surface antigen, said virus middle surface antigen or said virus large surface antigen each produced by the variant cell strain huGK-14. The column to which the monoclonal antibodies bind may be prepared by loading an IgG fraction of said monoclonal antibodies on a Sepharose column (Pharmacia AB) bonded by the CNBr activation method. The monoclonal antibodies binding to HBsAg would bind not only to the small surface antigen but also to those to which pre-S is attached, since these surface antigens have a common surface antigen polypeptide wherein the binding site with the antibody is present. Then a purified specimen of the small surface antigen, middle surface antigen and large surface antigen may be obtained by eluting the column with a phosphate buffer solution containing KSCN, concentrating antigen fractions, ultracentrifuging the antigen composition fraction by equilibrium centrifugation with the use of cesium chloride and passing the same through a filter membrane.

4. Immunogenicity Test

The specimen is subcutaneously injected into guinea pigs. Thus a high antibody titer is obtained (cf. Table 5 in Example 2), which suggests that it is a very excellent vaccine.

5. Isolation of small surface antigen from surface antigens having pre-S therein

The specimen comprises a large amount of the polypeptide and glycopolypeptide of the small surface antigen (two bands) and a smaller amount of the polypeptides and glycopolypeptides of the surface antigens having pre-S therein (four bands). The polypeptide and glycopolypeptide of the small surface antigen may be separated from those of the surface antigens in which pre-S is attached with the use of anti-pre-S antibody. The binding site of the anti-pre-S antibody with the antigen is present in 55 amino acids attached to the N-terminal side of the small surface antigen. Thus this antibody does not bind to the small surface antigen. The surface antigens having pre-S comprise middle surface antigen and large surface antigen both having a common sequence of 55 amino acids attached to the N-terminal side of the small surface antigen (cf. Fig. 5).

EXAMPLES

In order to illustrate the present invention, and not by way of limitation, the following Examples will be given.

EXAMPLE 1: Culture, recovery and purification

A DM-160 medium (mfd. by Kyokuto Seiyaku Co., Ltd.) containing 4 % of decomplemented fetal calf serum and 60 µg/l of kanamycin sulfate (mfd. by Meiji Seika Kaisha, Ltd.) was employed for the growth phase. The culture was carried out at 37°C and at a $CO_2$ concentration of 5 %. The variant cell strain huGK-14 was inoculated at a density of $2.5 \times 10^4$ cells/cm$^2$, into plastics Petri dishes (dia. 15 cm) and allowed to grow therein. Then cells in six plastic Petri dishes ($1.5 \times 10^8$ or more) were transferred into "Cell Factory" (mfd. by Nunc Co., Ltd.), which was a plastics multistep-adhesive culturing device having an adhesive area of 6000 cm$^2$, immediately before the cell density reached saturation ($1 \times 10^5$ cells/cm$^2$). The cells were allowed to grow therein. The inoculation density at each step was approximately $2.5 \times 10^4$ cells/cm$^2$ and transplantation was performed when it reached approximately $1 \times 10^5$ cells/cm$^2$. The conversion into a serum-free medium was carried out at the point when the cell density in the cell factory exceeded approximately $1 \times 10^5$ cells/cm$^2$. The serum-free medium was a Williams' E medium (mfd. by Flow Laboratories) containing $10^{-6}$ M of dexamethasone (mfd. by Sigma Co.), 60 µg/ml of kanamycin sulfate and an adhesive factor fibronectin (mfd. by Sigma Co.). The culture was continued under the abovementioned conditions while repeating the replacement of the medium at an interval of six days 15 times. Thus the supernatant was recovered.

The small surface antigen and surface antigens to which pre-S was attached in the culture supernatant thus obtained were determined by using an Orszyme HBsAg kit (mfd. by Dynabot). The amount of each antigen was calculated from a standard curve which showed the correlationship between an absorbancy at 492 nm and the amount of HBsAg determined by diluting a positive control. Table 2 shows the result.

Then purification was conducted using monoclonal antibodies.

Sodium azide at a concentration of 0.01 % was added to the culture supernatant thus recovered and then, the mixture was introduced into a continuous centrifuge (11500 x g, 10 min) to remove solid matters including cells, thus giving a supernatant.

The supernatant was concentrated in a circulating condensor (ultrafiltration; flat-membrane type, cut off molecular weight = 100,000, mfd. by Millipore Corp.) to give an approximately 30-fold concentrate. The concentrate was introduced into the continuous centrifuge (11500 xg, 10 min) again to remove insoluble matters and then subjected to a Sepharose 4B column (dia. 5 x 5.5 cm; mfd. by Pharmacia AB) to which an IgG fraction of monoclonal antibodies (prepared by K.K. Shino-test Laboratory) binding to the peptide moiety of the surface antigen was bound.

Then the column was washed with a phosphate buffer solution containing 0.5 M NaCl and eluted with a phosphate buffer solution containing 3 M KSCN. The eluate was dialyzed and introduced into an ultracentrifuge (130000 xg, 48 hrs) by equilibrium centrifugation with the use of CsCl. Then the antigen fraction was withdrawn, dialyzed against a buffer solution and filtered through a membrane of 0.22 μm to give a specimen. Thus a purified specimen of the surface antigen and those having surface antigen peptide moiety to which pre-S was attached was obtained. The purity at each step is shown in Table 2.

Table 2

| | Total protein(μg) | HBsAg antigen(μg) | yield (%) |
|---|---|---|---|
| Cell culture supernatant | —— | 1888.9 | 100 |
| Anti-HBsAg monoclonal antibody column | 2391.4 | 1795.4 | 95 |
| Ultracentrifugation (CsCl equilibrium centrifugation) | 1621.5 | 1363.8 | 72.2 |
| Filtration (0.22 μm) | 1644 | 779.3 | 41.3 |

Note: HBsAg includes the surface antigens with and without pre-S.

The purified specimen was then separated by means of SDS polyacrylamide gel electrophoresis according to the method of Laemmli et al. (cf. reference 25). The gel thus obtained was stained with silver according to the method of Oaklay et al. (cf. reference 26).

Bovine serum albumin of a molecular weight of 66,000, egg albumin of a molecular weight of 45,000 and β-lactoglobulin of a molecular weight of 18,400 were employed as standard proteins. The specimen showed six bands corresponding to molecular weights of 46,000, 43,000, 36,000, 31,000, 26,000 and 22,000 (cf. Fig. 2).

Among these bands, those corresponding to molecular weights of 26,000 and 22,000 were dense, showing major products, while those corresponding to molecular weights of 46,000, 43,000, 36,000 and 31,000 were relatively pale. Among the latter bands, the one of 36,000 was somewhat broad.

In order to prove that the difference in the molecular weights of the respective two polypeptides in each pair was caused by sugar chains, huGK-14 was cultured in a medium containing tunicamycin (mfd. by Sigma Co.) which inhibited the formation of sugar chains. huGK-14 was cultured in a medium containing 3 μg/ml of tunicamycin and the small surface antigen, middle surface antigen and large surface antigen were purified from the obtained culture medium. When the obtained specimen was subjected to SDS polyacrylamide gel electrophoresis, no bands corresponding to higher molecular weights were observed. That is, each pair was converted into a single band corresponding to the lower molecular weight. This fact suggests that the small surface antigen, middle surface antigen and large surface antigen comprised polypeptides and corresponding glycopolypeptides. Fig. 3 shows the major pair corresponding to molecular weights of 22,000 and 26,000. The band of 26,000 disappeared and a single band was observed at the position of 22,000.

Determination of the reactivity of the purified specimen as described above with anti-pre-S antibody has proved that it is a composition of the small surface antigen and those to which pre-S is attached. (This test was carried out by Atsuhiko Machida of Tokyo Prefectural Clinical Institute and Tadashi Mayumi of Jichi Medical College.)

The above test was carried out by reacting sheep erythrocytes treated with 5 % of tannic acid with a phosphate buffer solution containing anti-pre-S antibody at 37°C for two hours, successively diluting the reaction mixture twice with the use of a V-type microplate and subjecting 25 µl portions of the diluted specimen (50 µg/ml) to passive erythrocyte agglutination test (cf. references 24 and 27).

Consequently the specimen showed a positive result at an antigen titer with the anti-pre-S antibody of 1 : 4. Similarly the reactivity of the purified specimen (500 µg/ml) successively diluted twice with anti-HBs antibody was determined. As a result, the specimen exhibited an antibody titer of 1 : 128 showing an extremely high reactivity.

Thus the purified specimen was proved to be a composition of the small surface antigen and those to which pre-S was attached.

Table 4

Reactivity with          Reactivity with
anti-pre-S antibody   anti-HBs antibody

Purified specimen     1 : 4       1 : 128

EXAMPLE 2: Immunogenicity test as vaccine

The composition of the present invention was subcutaneously injected into guinea pigs to thereby examine the antibody productivity thereof.

1 ml portions of physiological saline solutions containing 12.8 µg and 25.6 µg of the purified specimen respectively were injected into healthy guinea pigs of 400 to 500 g in body weight thrice at an interval of two weeks. Blood of these animals was collected two weeks thereafter. The collected blood was agglutinated at room temperature and centrifuged to separate the serum. Thus samples for determination were obtained. Antibody titers of these samples were determined by passive erythrocyte agglutination test according to the method of Stavitsky et al. (cf. reference 27). Table 5 shows the result.

Thus the composition of the present invention showed extremely high antibody titers.

Table 5

|  | Injection dose | Antibody titer |
|---|---|---|
| Composition of Invention | 12.8 µg | 1 : 128 |
|  | 26.6 µg | 1 : 362 |

References

1. Blumberg et al.,

N.Y. Acad. Med., <u>40</u>, 377 (1964),

2. Blumberg et al.,

   JAMA, <u>191</u>, 540 (1965),

3. Prince et al.,

   Proc. Natl. Acad. Sci. USA, <u>60</u>, 814 (1968),

4. WHO Technical Report Series No. 570 "Viral Hepatitis" (1975),

5. Japanese Patent Application No. 149377/1982, Product Exhibiting Antigenicity of Hepatitis B Virus E Antigens and Process for the Production of Said Antigens

6. Notification No. 196 of the Ministry of Health and Welfare, "Precipitated Hepatitis B Vaccine", October 30, 1984,

7. Galibert et al.,

   Nature, <u>281</u>, 646 - 650 (1979),

8. Valenzuela et al.,

   Academic Press, 57 - 70 (1981),

9. Matsubara et al.,

   Nucleic Acids Research, <u>11</u>, 4601 - 4610 (1983),

10. Pasek et al.,

    Nature, <u>282</u>, 575 - 579 (1979),

11. Charnay et al.,

    Nature, <u>286</u>, 893 - 895 (1980),

12. Valenzuela et al.,

Nature, 298, 347 - 350 (1982),

13. Mcleer et al.,

    Nature, 307, 178 - 184 (1984),

14. Matsubara et al.,

    Proc. Natl. Acad. Sci. USA, 80, 1 - 5 (1983),

15. Japanese Patent Application No. 167950/1984, Vaccine and Composition against Hepatitis B Viral Infection,

16. Japanese Patent Application No. 173431/1984, Immunogenic HBsAg Derived from Transformed Yeast,

17. Japanese Patent Application No. 150627/1982, Production of Polypeptide in Vertebrate Cells,

18. Japanese Patent Application No. 191438/1982, DNA and Application of the Same,

19. Japanese Patent Application No. 148774/1983, Process for the Production of Hepatitis B Vaccine,

20. Koike et al.,

    Proc. Japan Acad., 58, 140 - 144 (1982),

21. Japanese Patent Application No. 142460/1982, Process for the Production of Recombined Plasmid, Yeast Transformed thereby and Hepatitis B Virus Surface Antigen,

22. Japanese Patent Application No. 183432/1982, Novel DNA, Process for the Production of the Same

0205625

- 26 -

and Transformant Containing the Same,

23. Mizusawa et al.,

Proc. Natl. Acad. Sci. USA, 82, 208-212 (1985),

24. Okamoto et al.,

J. of Immunology, 134 No. 2, 1212 - 1216 (1985),

25. Laemmli et al.,

Nature, 227, 6808 (1970),

26. Oakley et al.,

Anal. Biochem., 105, 361 (1980),

27. Stavitsky et al.,

Proc. Soc. Exp. Biol. Med., 124, 1166 (1967) and

28. Neurath et al.,

Nature, 315, 154 - 156 (1985).

What is claimed is:

(1) A composition which comprises at least one polypeptide and/or glycopolypeptide of adr hepatitis B virus small surface antigen and/or said virus middle surface antigen and/or said virus large surface antigen.

(2) A composition as set forth in Claim 1, wherein said small surface antigen exhibits an activity as hepatitis B virus surface antigen and has a molecular weight of 22,000 to 28,000.

(3) A composition as set forth in Claim 1, wherein said middle surface antigen exhibits an activity as hepatitis B virus antigen as well as a pre-S activity and has a molecular weight of 31,000 to 36,000.

(4) A composition as set forth in Claim 1, wherein said large surface antigen exhibits an activity as hepatitis B virus antigen as well as a pre-S activity and has a molecular weight of 43,000 to 47,000.

(5) A process for the production of a composition comprising at least one polypeptide and/or glycopolypeptide of adr hepatitis B virus small surface antigen and/or said virus middle surface antigen and/or said virus large surface antigen, which comprises culturing a cell strain huGK-14 originating from human hepatoma and recovering said composition from the culture product thus obtained.

(6) A hepatitis B vaccine containing a composition which comprises at least one polypeptide and/or glycopolypeptide of adr hepatitis B virus small surface antigen and/or said virus middle surface antigen and/or said virus large surface antigen as an active ingredient.

FIG. 1

0205625

2/6

FIG. 2

← BOVINE SERUM ALBUMIN

F →
E →
← EGG ALBUMIN
D →
C →

B →

A →

← β – LACTOGLOBULIN

MARKER PROTEIN
1. β – LACTOGLOBULIN 18,400 DALTONS
2. EGG ALBUMIN 45.000 DALTONS
3. BOVINE SERUM ALBUMIN 66.000 DALTONS

POLYPEPTIDE
A. 22.000 DALTONS
B. 26.000 DALTONS
C. 31.000 DALTONS
D. 36.000 DALTONS
E. 43.000 DALTONS
F. 46.000 DALTONS

# FIG. 3

LANE 1     LANE 2

26.000
22.000

22.000

LANE 1 : PURIFIED SMALL SURFACE ANTIGEN
LANE 2 : CRUDE SMALL SURFACE ANTIGEN
OBTAINED IN THE PRESENCE OF
TUNICAMYCIN (3μg/mℓ)

FIG. 4-(1)

```
         10        20        30        40        50        60        70        80        90       100
CTCGAGGACT GGGGACCCTG CACCGAACAT GGAGAGCACA ACATCAGGAT TCCTAGGACC CCTGCTCGTG TTACAGGCGG GGTTTTTCTT GTTGACAAGA

        110       120       130       140       150       160       170       180       190       200
ATCCTCACAA TACCACAGAG TCTAGACTCG TGGTGGACTT CTCTCAATTT TCTAGGGGGA GCACCCACGT GTCCTGGCCA AAATTCGCAG TCCCCAACCT

        210       220       230       240       250       260       270       280       290       300
CCAATCACTC ACCAACCTCT TGTCCTCCAA TTTGTCCTGG TTATCGCTGG ATGTGTCTGC GGCGTTTTAT CATATTCCTC TTCATCCTGC TGCTATGCCT

        310       320       330       340       350       360       370       380       390       400
CATCTTCTTG TTGGTTCTTC TGGACTACCA AGGTATGTTG CCCGTTTGTC CTCTACTTCC AGGAACATCA ACTACCAGCA CGGGACCATG CAAGACCTGC

        410       420       430       440       450       460       470       480       490       500
ACGATTCCTG CTCAAGGAAC CTCTATGTTT CCCTCTTGTT GCTGTACAAA ACCTTCGGAC GGAAACTGCA CTTGTATTCC CATCCCATCA TCCTGGGCTT

        510       520       530       540       550       560       570       580       590       600
TCGCAAGATT CCTATGGGAG TGGGCCTCAG TCCGTTTCTC CTGGCTCAGT TTACTAGTGC CATTTGTTCA GTGGTTCGCA GGGCTTTCCC CCACTGTTTG

        610       620       630       640       650       660       670       680       690       700
GCTTTCAGTT ATATGGATGA TGTGGTATTG GGGGCCAAGT CTGTACAACA TCTTGAGTCC CTTTTTACCT CTATTACCAA TTTTCTTTTG TCTTTGGGTA

        710       720       730       740       750       760       770       780       790       800
TACATTTGAA CCCTAATAAA ACCAAACGTT GGGGCTACTC CCTTAACTTC ATGGGATATG TAATTGGAAG TTGGGGTACT TTACCACAGG AACATATTGT

        810       820       830       840       850       860       870       880       890       900
ATTAAAACTC AAGCAATGTT TTCGAAAACT GCCTGTAAAT AGACCTATTG ATTGGAAAGT ATGTCAAAGA ATTGTGGGTC TTTTGGGCTT TGCTGCCCCT

        910       920       930       940       950       960       970       980       990      1000
TTTACACAAT GTGGCTATCC TGCCTTGATG CCTTTGTATG CATGTATACA ATCTAAGCAG GCTTTCACTT TCTCGCCAAC TTATAAGGCC TTTCTGTGTC

       1010      1020      1030      1040      1050      1060      1070      1080      1090      1100
AACAATACCT GCACCTTTAC CCCGTTGCCC GGCAACGGTC AGGTCTCTGC CAAGTGTTTG CTGACGCAAC CCCCACTGGA TGGGGCTTGG CCATAGGCCA

       1110      1120      1130      1140      1150      1160      1170      1180      1190      1200
TCGGCGCATG CGTGGAACCT TTGTGGTTCC TCTGCCGATC CATACTGCGG AACTCCTAGC AGCTTGTTTT GCTCGCGACC GGTCTGGAGC AAAACTTATC

       1210      1220      1230      1240      1250      1260      1270      1280      1290      1300
GGGACTGACA ACTCGGTTGT CCTCTCTCGG AAATACACCT CCTTCCCATG GCTGCTCGGG TGTGCTGCCA ACTGGATCCT GCGCGGGACG TCCTTTGTCT

       1310      1320      1330      1340      1350      1360      1370      1380      1390      1400
ACGTCCCGTC GGCGCTGAAT CCCGCGGACG ACCCGTCTCG GGGCCGTTTG GGCCTCTACC GTCCCTTGCT TTCTCTGCCG TTCCAGCCGA CCACGGGGCG

       1410      1420      1430      1440      1450      1460      1470      1480      1490      1500
CACCTCTCTT TACGCGGTCT CCCCGTCTGT GCCTTCTCAT CTGCCGGACC GTGTGCACTT CGCTTCACCT CTGCACGTCG CATGGAGACC ACCGTGAACG

       1510      1520      1530      1540      1550      1560      1570      1580      1590      1600
GCCACCAGGT CTTGCCCAAG CTCTTACATA AGAGGACTCT TGGACTCTCA GCAATGTCAA CAACCGACCT TGAGGCATAC TTCAAAGACT GTTTGTTTAA

       1610      1620      1630      1640      1650      1660      1670      1680      1690      1700
AGACTGGGAG GAGTTGGGGG AGGAGATTAG GTTAAAGGTC TTTGTACTAG GAGGCTGTAG GCATAAATTG GTCTGTTCAC CAGCACCATG CAACTTTTTC

       1710      1720      1730      1740      1750      1760      1770      1780      1790      1800
ACCTCTGCCT AATCATCTCA TGTTCATGTC CTACTGTTCA AGCCTCCAAG CTGTGCCTTG GGTGGCTTTG GGGCATGGAC ATTGACCCGT ATAAAGAATT

       1810      1820      1830      1840      1850      1860      1870      1880      1890      1900
TGGAGCTTCT GTGGAGTTAC TCTCTTTTTT GCCTTCTGAC TTCTTTCCTT CTATTCGAGA TCTCCTCGAC ACCGCCTCTG CTCTGTATCG GGAGGCCTTA

       1910      1920      1930      1940      1950      1960      1970      1980      1990      2000
GAGTCTCCGG AACATTGTTC ACCTCACCAT ACAGCACTCA GGCAAGCTAT TCTGTGTTGG GGTGAGTTGA TGAATTTGGC CACCTGGGTG GGAAGTAATT
```

4/6

FIG. 4-(2)

```
      2010        2020        2030        2040        2050        2060        2070        2080        2090        2100
TGGAAGACCC AGCATCCAGG GAATTAGTAG TCAGCTATGT CAATGTTAAT ATGGGCCTAA AAATCAGACA ACTATTGTGG TTTCATATTT CCTGTCTTAC

      2110        2120        2130        2140        2150        2160        2170        2180        2190        2200
TTTTGGAAGA GAAACTGTTC TTGAGTATTT GGTGTCTTTT GGAGTGTGGA TTCGCACTCC TCCCGCTTAC AGACCACCAA ATGCCCCTAT CTTATCAACA

      2210        2220        2230        2240        2250        2260        2270        2280        2290        2300
CTTCCGGAAA CTACTGTTGT TAGACGACGA GGCAGGTCCC CTAGAAGAAG AACTCCCTCG CCTCGCAGAC GAAGGTCTCA ATCGCCGCGT CGCAGAAGAT

      2310        2320        2330        2340        2350        2360        2370        2380        2390        2400
CTCAATCTCG GGAATCTCAA TGTTAGTATC CCTTGGACTC ATAAGGTGGG AAACTTTACT GGGCTTTATT CTTCTACTGT ACCTGTCCTT AATCCTGAGT

      2410        2420        2430        2440        2450        2460        2470        2480        2490        2500
GGCAAACTCC CTCCTTTCCT AACATTCATT TACAGGAGGA CATTATTAAT AGATGTCAAC AATATGTGGG CCCTCTTACA GTTAATGAAA AAAGGAGATT

      2510        2520        2530        2540        2550        2560        2570        2580        2590        2600
`AAAATTAATT ATGCCTGCTA GGTTCTATCC TAACCTTACC AAATATTTGC CCTTGGATAA AGGCATTAAA CCTTATTATC CTGAACATGC AGTTAATCAT

      2610        2620        2630        2640        2650        2660        2670        2680        2690        2700
TACTTCAAAA CTAGGCATTA TTTACATACT CTGTGGAAGG CTGGCATTCT ATATAAAAGA GAAACTACAC GCAGCGCTTC ATTTTGTGGG TCACCATATT

      2710        2720        2730        2740        2750        2760        2770        2780        2790        2800
CTTGGGAACA AGAGCTACGG CATGGGAGGT TGGTCTTCCA AACCTCGACA AGGCATGGGG ACGAATCTTT CTGTTCCCAA TCCTCTGGGA TTCTTTCCCG

      2810        2820        2830       ·2840        2850        2860        2870        2880        2890        2900
ATCACCAGTT GGACCCTGCG TTCGGAGCCA ACTCAAACAA TCCAGATTGG GACTTCAACC CCAACAAGGA TCACTGGCCA GAGGCAATCA AGGTAGGAGC

      2910        2920        2930        2940        2950        2960        2970        2980        2990        3000
GGGAGACTTC GGGCCAGGGT TCACCCCACC ACACGGCGGT CTTTTGGGGT GGAGCCCTCA GGCTCAGGGC ATATTGACAA CAGTGCCAGC AGCGCCTCCT

      3010        3020        3030        3040        3050        3060        3070        3080        3090        3100
CCTGTTTCCA CCAATCGGCA GTCAGGAAGA CAGCCTACTC CCATCTCTCC ACCTCTAAGA GACAGTCATC CTCAGGCCAT GCAGTGGAAC TCCACAACAT

      3110        3120        3130        3140        3150        3160        3170        3180        3190        3200
TCCACCAAGC TCTGCTAGAT CCCAGAGTGA GGGGCCTATA TTTTCCTGCT GGTGGCTCCA GTTCCGGAAC AGTAAACCCT GTTCCGACTA CTGTCTCACC

      3210        3220
CATATCGTCA ATCTT
```

1. _____ represents a base sequence of a gene coding for adr hepatitis B virus small surface antigen.
2. _____ + _____ represents a base sequence of a gene coding for adr hepatitis B virus middle surface antigen.
3. _____ + _____ + ____ represents a base sequence of a gene coding for adr hepatitis B virus large surface antigen.

0205625

# FIG. 5

| 1 | Met | Gly | Thr | Asn | Leu | Ser | Val | Pro | Asn | Pro | 10 |
| 11 | Leu | Gly | Phe | Phe | Pro | Asp | His | Gln | Leu | Asp | 20 |
| 21 | Pro | Ala | Phe | Gly | Ala | Asn | Ser | Asn | Asn | Pro | 30 |
| 31 | Asp | Trp | Asp | Phe | Asn | Pro | Asn | Lys | Asp | His | 40 |
| 41 | Trp | Pro | Glu | Ala | Ile | Lys | Val | Gly | Ala | Gly | 50 |
| 51 | Asp | Phe | Gly | Pro | Gly | Phe | Thr | Pro | Pro | His | 60 |
| 61 | Gly | Gly | Leu | Leu | Gly | Trp | Ser | Pro | Gln | Ala | 70 |
| 71 | Gln | Gly | Ile | Leu | Thr | Thr | Val | Pro | Ala | Ala | 80 |
| 81 | Pro | Pro | Pro | Val | Ser | Thr | Asn | Arg | Gln | Ser | 90 |
| 91 | Gly | Arg | Gln | Pro | Thr | Pro | Ile | Ser | Pro | Pro | 100 |
| 101 | Leu | Arg | Asp | Ser | His | Pro | Gln | Ala | Met | Gln | 110 |
| 111 | Trp | Asn | Ser | Thr | Thr | Phe | His | Gln | Ala | Leu | 120 |
| 121 | Leu | Asp | Pro | Arg | Val | Arg | Gly | Leu | Tyr | Phe | 130 |
| 131 | Pro | Ala | Gly | Gly | Ser | Ser | Ser | Gly | Thr | Val | 140 |
| 141 | Asn | Pro | Val | Pro | Thr | Thr | Val | Ser | Pro | Ile | 150 |
| 151 | Ser | Ser | Ile | Phe | Ser | Arg | Thr | Gly | Asp | Pro | 160 |
| 161 | Ala | Pro | Asn | Met | Glu | Ser | Thr | Thr | Ser | Gly | 170 |
| 171 | Phe | Leu | Gly | Pro | Leu | Leu | Val | Leu | Gln | Ala | 180 |
| 181 | Gly | Phe | Phe | Leu | Leu | Thr | Arg | Ile | Leu | Thr | 190 |
| 191 | Ile | Pro | Gln | Ser | Leu | Asp | Ser | Trp | Trp | Thr | 200 |
| 201 | Ser | Leu | Asn | Phe | Leu | Gly | Gly | Ala | Pro | Thr | 210 |
| 211 | Cys | Pro | Gly | Gln | Asn | Ser | Gln | Ser | Pro | Thr | 220 |
| 221 | Ser | Asn | His | Ser | Pro | Thr | Ser | Cys | Pro | Pro | 230 |
| 231 | Ile | Cys | Pro | Gly | Tyr | Arg | Trp | Met | Cys | Leu | 240 |
| 241 | Arg | Arg | Phe | Ile | Ile | Phe | Leu | Phe | Ile | Leu | 250 |
| 251 | Leu | Leu | Cys | Leu | Ile | Phe | Leu | Leu | Val | Leu | 260 |
| 261 | Leu | Asp | Tyr | Gln | Gly | Met | Leu | Pro | Val | Cys | 270 |
| 271 | Pro | Leu | Leu | Pro | Gly | Thr | Ser | Thr | Thr | Ser | 280 |
| 281 | Thr | Gly | Pro | Cys | Lys | Thr | Cys | Thr | Ile | Pro | 290 |
| 291 | Ala | Gln | Gly | Thr | Ser | Met | Phe | Pro | Ser | Cys | 300 |
| 301 | Cys | Cys | Thr | Lys | Pro | Ser | Asp | Gly | Asn | Cys | 310 |
| 311 | Thr | Cys | Ile | Pro | Ile | Pro | Ser | Ser | Trp | Ala | 320 |
| 321 | Phe | Ala | Arg | Phe | Leu | Trp | Glu | Trp | Ala | Ser | 330 |
| 331 | Val | Arg | Phe | Ser | Trp | Leu | Ser | Leu | Leu | Val | 340 |
| 341 | Pro | Phe | Val | Gln | Trp | Phe | Ala | Gly | Leu | Ser | 350 |
| 351 | Pro | Thr | Val | Trp | Leu | Ser | Val | Ile | Trp | Met | 360 |
| 361 | Met | Trp | Tyr | Trp | Gly | Pro | Ser | Leu | Tyr | Asn | 370 |
| 371 | Ile | Leu | Ser | Pro | Phe | Leu | Pro | Leu | Leu | Pro | 380 |
| 381 | Ile | Phe | Phe | Cys | Leu | Trp | Val | Tyr | Ile | | |

1.  ————————— represents an amino acid sequence of adr hepatitis B virus small surface antigen.

2.  ————— + ————— represents an amino acid sequence of adr hepatitis B virus middle surface antigen.

3.  ————— + ————— + ———— represents an amono acid sequence of adr hepatitis B virus large surface antigen.

# INTERNATIONAL SEARCH REPORT

0205625

International Application No PCT/JP85/00733

## I. CLASSIFICATION OF SUBJECT MATTER (if several classification symbols apply, indicate all) [3]

According to International Patent Classification (IPC) or to both National Classification and IPC

Int.Cl$^4$   A61K 39/29, C07K 15/04, C12P 21/00

## II. FIELDS SEARCHED

### Minimum Documentation Searched [4]

| Classification System | Classification Symbols |
|---|---|
| IPC | A61K 39/29, C07K 15/04, C12P 21/00 |

Documentation Searched other than Minimum Documentation
to the Extent that such Documents are Included in the Fields Searched [5]

## III. DOCUMENTS CONSIDERED TO BE RELEVANT [14]

| Category* | Citation of Document, [16] with indication, where appropriate, of the relevant passages [17] | Relevant to Claim No. [18] |
|---|---|---|
| A | JP, A, 56-150020 (Merck & Co., Inc.), 20 November 1981 (20. 11. 81) & EP, A, 37330 | 5 |
| Y | JP, A, 51-139619 (Merck & Co., Inc.), 2 December 1976 (02. 12. 76) & DE, A, 2621276 & FR, A, 2315947 & US, A, 4017360 | 1-4, 6 |
| Y | JP, A, 59-74985 (Takeda Chemical Industries, Ltd.), 27 April 1984 (27. 04. 84) (Family: none) | 1-4, 6 |
| Y | Chemical Abstracts, Vol. 94, No.11, 16 March 1981 (16. 03. 81) (Columbus, Ohio, U.S.A.), p.542 Abstract No. 81924v | 1-4, 6 |

* Special categories of cited documents: [16]

"A" document defining the general state of the art which is not considered to be of particular relevance

"E" earlier document but published on or after the international filing date

"L" document which may throw doubts on priority claim(s) or which is cited· to establish the publication date of another citation or other special reason (as specified)

"O" document referring to an oral disclosure. use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search [2] | Date of Mailing of this International Search Report [2] |
|---|---|
| March 24, 1986 (24. 03. 86) | April 7, 1986 (07. 04. 86) |
| International Searching Authority | Signature of Authorized Officer [70] |
| Japanese Patent Office | |

Form PCT/ISA/210 (second sheet October 1981)